Europäisches Patentamt

(19) European Patent Office

Office européen des brevets

(11) EP 0 757 030 B1

(12) EUROPEAN PATENT SPECIFICATION

(45) Date of publication and mention
of the grant of the patent:
**03.11.1999 Bulletin 1999/44**

(51) Int. Cl.$^6$: **C07C 68/08**, C07C 69/96

(21) Application number: **96305303.8**

(22) Date of filing: **19.07.1996**

(54) **Process for purifying diaryl carbonates**

Verfahren zur Reinigung von Diarylcarbonaten

Procédé de purification de diarylcarbonates

(84) Designated Contracting States:
**BE DE ES FR GB IT NL**

(30) Priority: **01.08.1995 US 509891**

(43) Date of publication of application:
**05.02.1997 Bulletin 1997/06**

(73) Proprietor:
**GENERAL ELECTRIC COMPANY
Schenectady, NY 12345 (US)**

(72) Inventor:
**Kissinger, Gaylord Michael
Evansville, Indiana 47711 (US)**

(74) Representative:
**Szary, Anne Catherine, Dr.
London Patent Operation,
GE International, Inc.,
Essex House,
12-13 Essex Street
London WC2R 3AA (GB)**

(56) References cited:
**EP-A- 0 583 936          EP-A- 0 633 241
EP-A- 0 636 620          EP-A- 0 687 666**

## Description

The invention relates to processes for the purification of diaryl carbonates such as diphenyl carbonate.

## Brief Description of Related Art

Diaryl carbonates such as diphenyl carbonate may be prepared by a number of processes. For example, one process is based on the phosgenation of aromatic hydroxy compounds in the presence of a quaternary ammonium salt catalyst. A variant process comprises phosgenation at elevated temperatures in alkaline solution. A commercial process for production of diphenyl carbonate comprises phosgenation of phenol in the presence of caustic at a pH of 10-11 and at a temperature of 55°-60°C.

Commercial preparations of diaryl carbonates, regardless of the process employed, inevitably contain various contaminant compounds in varying quantities. Examples of contaminants which have been identified are inorganic and organic chlorides, metal ions, iron compounds, acidic compounds such as aryl chloroformates and a range of compounds identified only as "color bodies". These contaminants frequently effect use of the diaryl carbonates in particular applications. For example, when their desired use is as a monomer reactant in the preparation of aromatic polycarbonate resins by transesterification (using bisphenol-A and diphenyl carbonate), presence of the contaminants can affect polymerization rates and resin color. The polymer product may have a low intrinsic viscosity (IV) and colors ranging from pink (iron contamination) to brown (phenyl chloroformate contamination).

Although distillation of diaryl carbonates has been useful to remove color bodies and solves the problem of coloration in the carbonate and resins prepared from them, the procedure has not been useful to remove other contaminants responsible for inhibiting polymerization rates. In fact, a major drawback of distilled diphenyl carbonate is a loss of reactivity as observed in ester interchange reaction studies. The reason is open to speculation. Also, subjecting the diaryl carbonate, such as diphenyl carbonate, to distillation solely as a means of purification results in a percentage of product loss, which is an economic disadvantage.

Relatively crude diaryl carbonate containing contaminants associated with the process of preparation may be purified advantageously by a two-stage procedure to optimize overall yield of the desired product and remove contaminants. The first stage is washing a melt of the diaryl carbonate with water; followed by a second distillation stage. An intermediate cut taken during distillation between the first and last (product) cuts helps to isolate color bodies and contaminants responsible for producing low intrinsic viscosity and/or colored polymer resins therefrom. The distilled product is of improved reactivity and high purity, when the distillation procedure is immediately preceded by a hot aqueous wash. Water washing of the crude diaryl carbonate helps reduce the content of contaminants including iron, and improves final color in polymers prepared from the purified diaryl carbonate.

EP-A-0687666 discloses a process for the purification of diphenyl carbonate (DPC) by crystallization from crude products of diphenyl carbonate production which have diphenyl carbonate contents of above 70 wt. %, relative to the distillable fraction, by fractionating melt crystallization in which the melt to be purified is cooled in the range from 85°C to 45°C, at a cooling rate of 20° to 0.1°C/h, a holding time of 0 to 100 minutes is maintained at the lowest coolant temperature before separation of the residual melt, the crystallized product is then melted by heating at a heating rate of 20 to 0.1°C/h to a final temperature of 70° to 80°C and, during heating, further fractions of the melt with impurities are separated at pauses or without interruption of heating from the pure DPC melt arising at a higher temperature.

I have found that diaryl carbonates can also be readily separated and purified by crystallization from molten crude feedstocks. The crystallizations may be carried out by both falling film crystallization, and static melt crystallization methods. In principle, both these methods are fundamentally the "same", in that crystals, rich in the desired product are caused to separate from the impure melt by carefully and slowly lowering the melt temperature. Carefully lowering the temperature from the point where nucleation occurs, until a certain desired low temperature is reached, causes the liquid to be continuously saturated with the dominant component, thereby resulting in the formation of a solid phase (crystals), rich in the desired diaryl carbonate.

The process of the invention may be carried out to obtain pure diaryl carbonates in a continuous or a batch procedure, with an advantage in that the product is consistently obtained in high purity.

## SUMMARY OF THE INVENTION

The invention comprises a process for purification of diaryl carbonates, which comprises;

providing a crude solution of diaryl carbonate in admixture with contaminant by-products of the diaryl carbonate preparation;
cooling the solution to a temperature of 1-2°C. below the nucleation temperature of the diaryl carbonate, whereby nucleation occurs;

cooling the solution containing nucleated diaryl carbonate at a controlled rate, between 0.01 to 1.0°C. per minute whereby crystals of the diaryl carbonate form in a residue of cooled solution;

separating the residue of solution from the formed crystals of the diaryl carbonate;

heating the separated crystals at a controlled rate to their melt temperature, incrementally;

separating sweat exuding from the heated crystals in each increment; and

collecting the melted crystals to obtain high purity diaryl carbonate.

Near theoretical yields of the diaryl carbonate can be recovered in a semi-continuous embodiment process of the invention whereby the separated sweats and solution residues are recycled through the process for repeated fractional melt crystallization.

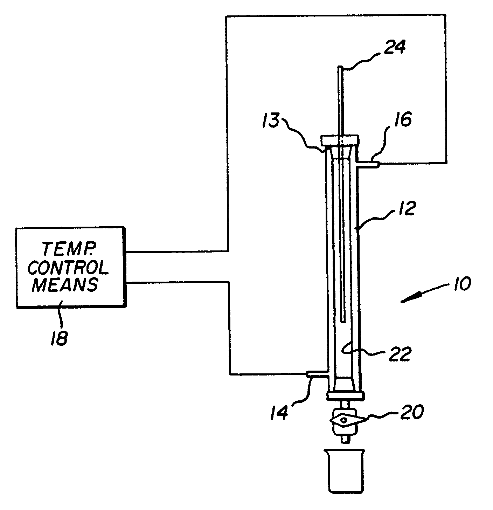

## BRIEF DESCRIPTION OF THE DRAWING

The accompanying drawing is a schematic showing a preferred embodiment apparatus for carrying out the process of the invention.

## DETAILED DESCRIPTION OF THE PREFERRED EMBODIMENTS OF THE INVENTION

The following description will be directed to a preferred embodiment process of the invention for purification of diphenyl carbonate (DPC). Those skilled in the art will appreciate that the inventive process is applicable to purification of other diaryl carbonates, such as for example, di-ortho-cresyl carbonate, di-ortho-chlorophenyl carbonate and the like.

With reference to the accompanying drawing, there is seen a schematic drawing of a static crystallizer assembly 10 for carrying out the purification of a diaryl carbonate contained in a crude product obtained for example by phosgenation of a phenol reactant compound.

A portion of melted crude diphenyl carbonate (DPC) feedstock obtained as a product of the preparation is added through the top end (13) of crystallizer jacketed tube (12). After precooling, the temperature of the crude melt is held at a constant temperature, 1-2°C. below the nucleation temperature of the DPC in the crude mixture. Once nucleation has occurred, the temperature is ramped downward slowly at a controlled rate, typically between about 0.01-1.0°C. per minute by circulating a heat transfer medium such as cooling water through the jacketed tube 12 via inlet 14 and outlet 16. Temperature control means 18 maintains a desired temperature ramp and control of the temperature in crystallizer assembly 10 and can be verified by thermometer (24). Crystallization proceeds as the cooling feedstock liquid continues to saturate, forming crystals which attach to the interior wall surface (22) of the crystallizer (10).

When the desired lowest temperature is reached, the bottom valve (20) is opened, and the uncrystallized liquid feedstock residue containing concentrated impurities is drained away from the crystallizer (10).

After the feedstock residue is separated, the temperature of crystallizer (10) is raised incrementally at a controlled rate to bring about a "sweating" or partial melting of the collected crystals. The optimal sweating temperature ramp, as well as the initial crystallization temperature ramp, are determined experimentally by trial and error technique to maximize purification per increment or stage, and to maximize the overall yield of the multi-stage process. Thus in a preferred embodiment process of the invention, residues and sweat liquors obtained from higher purity stages are "added back" to the feed of lower purity stages, until a purified product is taken from the highest yield stage, and residue for recycling in the process is taken from the lowest yield stage.

Table 1 below is a mass distribution diagram of one stage of the process of the invention with a static crystallization. One can see that substantial purification of the desired product (DPC), is accomplished by observing the DPC content of the feedstock (53.7% DPC) vs the melt product (96.7% DPC). It is obvious that the process using multiple stages will lead to very high purity.

Determination of the number of stages required to reach a predetermined purity and color, and the overall yield and efficiency may be derived by trial and error technique.

The following example describes the manner and process of carrying out the invention and sets forth the best mode contemplated by the inventor, but is not to be construed as limiting the scope of the invention.

## EXAMPLE

334 grams of crude diphenylcarbonate (DPC) feed (53.7% DPC) was melted and placed in the static crystallizer (10). The temperature was cooled quickly to around 53°C. Nucleation occurred at 53.8°C. The cooling ramp was started at 0.13 degrees per minute, and continued until it reached 30°C. At this point, the bottom valve (20) was opened, and the liquid residue allowed to drain for approximately 1 hour. The temperature was then raised in 10°C. increments, and held for one hour at each level while the "sweat" was collected. finally, after collecting the 75°C. sweat, the temperature was raised to 85°C. to quickly melt the product from one stage. All the fractions, residues and the starting solution were analyzed by high pressure liquid chromatography (HPLC) to determine the DPC content of each, and therefore the mass distribution of recovered DPC which is shown in Table 1, below.

TABLE 1

|  | Weight (gms) | DPC (%) | DPC (Grams) |
|---|---|---|---|
| Crude Starting Solution | 333.55 | 53.74 | 179.3 |
| Residue Removed after crystallization | 89.67 | 20.32 | 18.2 |
| Sweat 1 | 41.04 | 21.12 | 8.7 |
| Sweat 2 | 25.76 | 32.59 | 8.4 |
| Sweat 3 | 26.41 | 32.59 | 8.6 |
| Sweat 4 | 44.66 | 72.93 | 32.6 |
| Sweat 5 | 32.4 | 86.56 | 28.1 |
| Melt | 73.4 | 96.67 | 71.0 |
|  | Total Recovered | | 175.6 |

In a preferred embodiment process of the invention, the sweats 4 and 5 would be added to the residue after crystallization and mixed with or used as the crude starting solution in a subsequent multi-stage processing in order to recover a maximum yield of the desired DPC product.

**Claims**

**Claims for the following Contracting States : BE, DE, FR, GB, IT, NL**

1.   A process for purification of diaryl carbonates, which comprises;

providing a crude solution of diaryl carbonate in admixture with contaminant by-products of a diaryl carbonate preparation;
cooling the solution to a temperature of 1-2°C. below the nucleation temperature of the diaryl carbonate whereby nucleation occurs;
cooling the solution containing nucleated diaryl carbonate at a controlled rate, between 0.01 to 1.0°C. per minute whereby crystals of the diaryl carbonate form in a residue of cooled solution;
separating the residue of cooled solution from the formed crystals of the diaryl carbonate;
heating the separated crystals at a controlled rate to their melt temperature, incrementally;
separating sweat exuding from the heated crystals in each increment; and
collecting the melted crystals to obtain high purity diaryl carbonate; wherein the sweats are recycled into the residue for repeating the process or wherein the residue is recycled into the crude solution in a continuous process.

2.   The process of claim 1 wherein the diaryl carbonate is diphenyl carbonate.

3.   The process of claim 2 wherein the residue is employed as the crude solution.

4.   The process of claim 1 wherein the controlled rate of heating is in increments of 10°C and each 10°C increment is maintained for 1 hour.

**Claims for the following Contracting State : ES**

1.   A process for purification of diaryl carbonates, which comprises;

providing a crude solution of diaryl carbonate in admixture with contaminant by-products of a diaryl carbonate preparation;
cooling the solution to a temperature of 1-2°C. below the nucleation temperature of the diaryl carbonate

whereby nucleation occurs;

cooling the solution containing nucleated diaryl carbonate at a controlled rate, between 0.01 to 1.0°C. per minute whereby crystals of the diaryl carbonate form in a residue of cooled solution;

separating the residue of cooled solution from the formed crystals of the diaryl carbonate;

heating the separated crystals at a controlled rate to their melt temperature, incrementally;

separating sweat exuding from the heated crystals in each increment; and

collecting the melted crystals to obtain high purity diaryl carbonate.

2. The process of claim 1 wherein the diaryl carbonate is diphenyl carbonate.

3. The process of claim 1 wherein the sweats are recycled into the residue for repeating the process of claim 1.

4. The process of claim 1 wherein the residue is recycled into the crude solution in a continous process.

5. The process of claim 4 wherein the residue is employed as the crude solution.

6. The process of Claim 1 wherein the controlled rate of heating is in increments of 10°C and each 10°C increment is maintained for 1 hour.

**Patentansprüche**

**Patentansprüche für folgende Vertragsstaaten : BE, DE, FR, GB, IT, NL**

1. Verfahren zur Reinigung von Diarylcarbonaten aufweisend

das Bereitstellen einer Rohlösung eines Diarylcarbonats in Mischung mit verunreinigenden Nebenprodukten der Diarylcarbonatherstellung,

das Kühlen der Lösung auf eine Temperatur von 1-2°C unterhalb der Nukleierungstemperatur des Diarylcarbonats, wodurch Keimbildung auftritt,

das Kühlen der Lösung, die keimwirkendes Diarylcarbonat enthält, mit einer gesteuerten Geschwindigkeit von 0,01-1°C pro Minute, wodurch Kristalle des Diarylcarbonats aus einem Rückstand der gekühlten Lösung gebildet werden,

das Abtrennen des Rückstands der gekühlten Lösung von den gebildeten Diarylcarbonatkristallen,

das inkrementelle Erwärmen der abgetrennten Kristalle mit einer gesteuerten Geschwindigkeit auf ihre Schmelztemperatur,

das Abtrennen des Kondensats, das sich aus den erwärmten Kristallen in jedem Inkrement abscheidet und

das Sammeln der geschmolzenen Kristalle, um hoch reines Diarylcarbonat zu erhalten, wobei das Kondensat jeweils in den Rückstand geleitet wird, um das Verfahren zu wiederholen oder, wobei der Rückstand in die Rohlösung in einem kontinuierlichen Prozeß zugeführt wird.

2. Verfahren gemäß Anspruch 1, dadurch gekennzeichnet, daß das Diarylcarbonat Diphenylcarbonat ist.

3. Verfahren gemäß Anspruch 2, dadurch gekennzeichnet, daß der Rest als Rohlösung eingesetzt wird.

4. Verfahren gemäß Anspruch 1, dadurch gekennzeichnet, daß die gesteuerte Erwärmungsgeschwindigkeit in Inkrementen von 10°C stattfindet und jedes 10°C Inkrement für eine Stunde aufrecht erhalten wird.

**Patentansprüche für folgenden Vertragsstaat : ES**

1. Verfahren zur Reinigung von Diarylcarbonaten aufweisend

das Bereitstellen einer Rohlösung eines Diarylcarbonats in Mischung mit verunreinigenden Nebenprodukten der Diarylcarbonatherstellung,

das Kühlen der Lösung auf eine Temperatur von 1-2°C unterhalb der Nukleierungstemperatur des Diarylcarbonats, wodurch Keimbildung auftritt,

das Kühlen der Lösung, die keimwirkendes Diarylcarbonat enthält, mit einer gesteuerten Geschwindigkeit von 0,01-1°C pro Minute, wodurch Kristalle des Diarylcarbonats aus einem Rückstand der gekühlten Lösung gebildet werden,

das Abtrennen des Rückstands der gekühlten Lösung von den gebildeten Diarylcarbonatkristallen,

das inkrementelle Erwärmen der abgetrennten Kristalle mit einer gesteuerten Geschwindigkeit auf ihre Schmelztemperatur,

das Abtrennen des Kondensats, das sich aus den erwärmten Kristallen in jedem Inkrement abscheidet und das Sammeln der geschmolzenen Kristalle, um hoch reines Diarylcarbonat zu erhalten.

2. Verfahren gemäß Anspruch 1, dadurch gekennzeichnet, daß das Diarylcarbonat Diphenylcarbonat ist.

3. Verfahren gemäß Anspruch 1, dadurch gekennzeichnet, daß das Kondensat jeweils in den Rückstand geleitet wird, um das Verfahren zu wiederholen.

4. Verfahren gemäß Anspruch 1, dadurch gekennzeichnet, daß der Rückstand in die Rohlösung in einem kontinuierlichen Prozeß zugeführt wird.

5. Verfahren gemäß Anspruch 4, dadurch gekennzeichnet, daß der Rückstand als Rohlösung eingesetzt wird.

6. Verfahren gemäß Anspruch 1, dadurch gekennzeichnet, daß die gesteuerte Erwärmungsgeschwindigkeit in Inkrementen von 10°C stattfindet und jedes 10°C Inkrement für eine Stunde aufrecht erhalten wird.

## Revendications

**Revendications pour les Etats contractants suivants : BE, DE, FR, GB, IT, NL**

1. Procédé pour purifier des carbonates de diaryle, qui comprend :

le fait de fournir une solution brute de carbonate de diaryle mélangé avec des produits secondaires de type impureté d'une préparation de carbonate de diaryle ;
le fait de refroidir la solution jusqu'à une température inférieure de 1 à 2°C à la température de nucléation du carbonate de diaryle, à laquelle des germes apparaîssent ;
le fait de refroidir la solution contenant le carbonate de diaryle formant des germes à une vitesse réglée, entre 0,01 et 1,0°C par minute, les cristaux de carbonate de diaryle se formant ainsi dans un résidu de solution refroidie ;
le fait de séparer le résidu de solution refroidie des cristaux formés du carbonate de diaryle ;
le fait de chauffer les cristaux séparés à une vitesse réglée jusqu'à leur température de fusion, par paliers ;
le fait de séparer le liquide de ressuage suintant des cristaux chauffés, à chaque palier ; et
le fait de récupérer les cristaux fondus pour obtenir du carbonate de diaryle de grande pureté ; dans lequel le liquide de ressuage est recyclé dans le résidu pour répéter le procédé, ou bien dans lequel le résidu est recyclé dans la solution brute selon un procédé continu.

2. Procédé selon la revendication 1, dans lequel le carbonate de diaryle est le carbonate de diphényle.

3. Procédé selon la revendication 2, dans lequel le résidu est employé comme solution brute.

4. Procédé selon la revendication 1, dans lequel le chauffage à vitesse réglée se fait par paliers de 10°C en 10°C et chacun de ces paliers est maintenu pendant 1 heure.

**Revendications pour l'Etat contractant suivant : ES**

1. Procédé pour purifier des carbonates de diaryle, qui comprend :

le fait de fournir une solution brute de carbonate de diaryle mélangé avec des produits secondaires de type impureté d'une préparation de carbonate de diaryle ;
le fait de refroidir la solution jusqu'à une température inférieure de 1 à 2°C à la température de nucléation du carbonate de diaryle, à laquelle des germes apparaîssent ;
le fait de refroidir la solution contenant le carbonate de diaryle formant des germes à une vitesse réglée, entre 0,01 et 1,0°C par minute, des cristaux de carbonate de diaryle se formant ainsi dans un résidu de solution refroidie ;
le fait de séparer le résidu de solution refroidie des cristaux formés du carbonate de diaryle ;

le fait de chauffer les cristaux séparés à une vitesse réglée jusqu'à leur température de fusion, par paliers ;

le fait de séparer le liquide de ressuage suintant des cristaux chauffés, à chaque palier ; et

le fait de récupérer les cristaux fondus pour obtenir du carbonate de diaryle de grande pureté.

2. Procédé selon la revendication 1, dans lequel le carbonate de diaryle est le carbonate de diphényle.

3. Procédé selon la revendication 1, dans lequel le liquide de ressuage est recyclé dans le résidu pour répéter le procédé de la revendication 1.

4. Procédé selon la revendication 1, dans lequel le résidu est recyclé dans la solution brute selon un procédé continu.

5. Procédé selon la revendication 4, dans lequel le résidu est employé comme solution brute.

6. Procédé selon la revendication 1, dans lequel e chauffage à vitesse réglée se fait par paliers de 10°C en 10°C et chacun de ces paliers est maintenu pendant 1 heure.

EP 0 757 030 B1

8